# FASCICULE DE BREVET EUROPEEN

(11) **EP 0 534 217 B1**
(45) Date de publication et mention de la délivrance du brevet: **31.01.1996**
(21) Numéro de dépôt: 92115249.2
(22) Date de dépôt: 07.09.1992
(51) Int. Cl.: A61B 8/08, G01S 15/50

(54) **Procédé de mesure par ultrasons de la position d'au moins une interface mobile et appareil pour la mise en oeuvre de ce procédé**
Ultraschall-Messverfahren für die Lagebestimmung von mindestens einer mobilen Schnittstelle und Gerät zur Anwendung des Verfahrens
Process for ultrasonic measurement of the position of at least one moving interface and means for application of the process

(30) Priorité: 27.09.1991 CH 2871/91; 04.10.1991 FR 9112338
(43) Date de publication de la demande: 31.03.1993
(73) Titulaire: ASULAB S.A., CH-2501 Bienne (CH)
(72) Inventeur: Mignot, Jean-Pierre, CH-2034 Peseux (CH)
(74) Mandataire: Thérond, Gérard Raymond

(56) Documents cités:
- DE-A- 2 853 170
- FR-A- 2 632 733
- US-A- 4 370 985

## Description

La présente invention concerne un procédé de mesure par ultrasons de la position d'au moins une paroi mobile et un appareil pour la mise en oeuvre de ce procédé.

L'invention trouve une application dans tous les domaines dans lesquels on souhaite suivre l'évolution temporelle de la position d'une paroi mobile, et notamment dans le domaine médical. Dans ce dernier, l'invention peut être utilisée pour suivre l'évolution temporelle de la position des interfaces des parois antérieure et postérieure d'un vaisseau sanguin de manière à déterminer l'évolution temporelle du diamètre intérieur et de l'épaisseur des parois de ce vaisseau sanguin. Elle peut également trouver application dans la mesure de l'épaisseur de la lentille cornéenne.

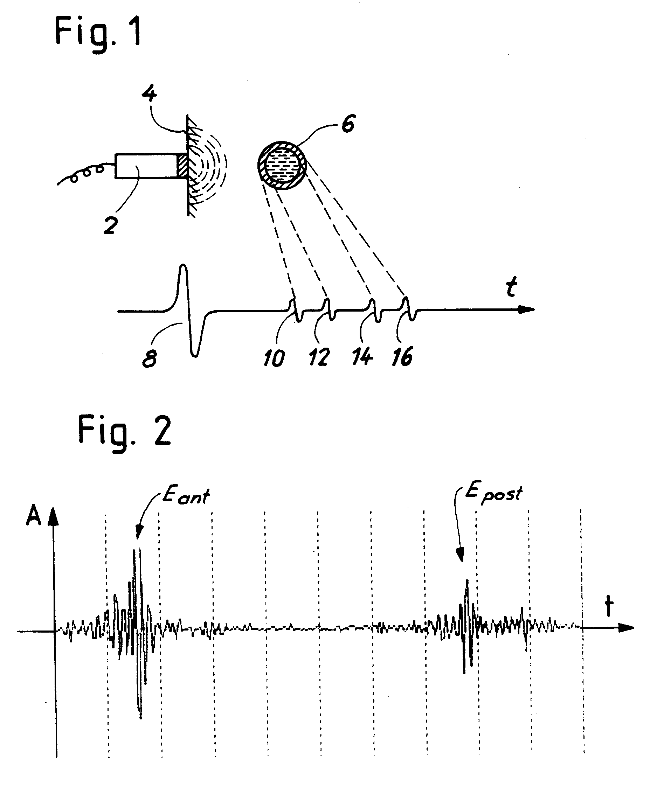

La figure 1 illustre schématiquement le principe de la mesure du déplacement d'au moins une paroi mobile. Cette figure représente un transducteur ultrasonore 2 placé sur la peau 4 d'un sujet en face d'une artère 6 représentée en coupe transversale. Le transducteur 2 est commandé par un circuit électronique pour émettre une impulsion d'onde ultrasonore 8 et pour recevoir les échos résultant de la réflexion de cette impulsion sur les interfaces artère-tissu ou artère-sang. Selon la fréquence du transducteur ultrasonore, on peut détecter quatre échos distincts 10, 12, 14, 16 ou seulement deux échos correspondant respectivement à une combinaison des échos 10 et 12, et à une combinaison des échos 14 et 16.

La connaissance de la position de chaque interface en fonction du temps, ainsi que la vitesse de propagation du son dans le sang et les tissus permettent, par différence, de déterminer l'évolution temporelle du diamètre intérieur et de l'épaisseur des parois antérieure et postérieure du vaisseau sanguin 6 en fonction du temps.

La figure 1 est un schéma de principe. En pratique, les échos Eₐₙₜ et Eₚₒₛₜ formés par les parois antérieure et postérieure du vaisseau sanguin ne sont pas aussi purs, mais présentent une forme beaucoup plus complexe comme représenté sur la figure 2. Cette déformation résulte de ce que le signal ultrasonore traverse des tissus de différentes natures et de ce que l'interface entre la paroi d'un vaisseau sanguin et le tissu environnant n'est pas parfaitement définie.

La position d'une interface, dans le domaine médical notamment, n'est donc pas déductible directement et automatiquement de la forme du signal d'écho.

Plusieurs techniques de détection par ultrasons de la position d'une interface mobile sont connues.

Une première technique consiste à traiter le signal d'écho pour supprimer les bruits et ne conserver pratiquement que la partie du signal résultant de la réflexion du signal ultrasonore sur l'interface. Cette technique présente cependant l'inconvénient de ne pas pouvoir être réalisée en temps réel. En effet, avec des moyens de calcul conventionnels, le traitement du signal d'écho demande de l'ordre de 0,1 à 5 secondes alors que, avec une fréquence de répétition de 100 Hz, le temps disponible pour traiter en temps réel un signal d'écho est de l'ordre de 0,01 seconde.

Il faut donc procéder en deux étapes : dans un premier temps, mémoriser en temps réel l'ensemble des signaux d'écho à étudier et, dans un second temps, traiter ces signaux d'écho. On conçoit que cette technique présente trois inconvénients qui sont la nécessité de disposer d'un volume de mémoire élevé, la durée de traitement des signaux d'écho et l'absence de contrôle en temps réel des données en cours d'acquisition.

Selon une seconde technique connue, la position de l'interface est déterminée de manière manuelle. L'utilisateur envoie le signal d'écho sur un oscilloscope ou tout autre moyen d'affichage et choisit un point particulier du signal d'écho, sur lequel le suiveur d'écho doit se caler. Les inconvénients de la première technique sont supprimés. En revanche, cette technique requiert de la part de l'utilisateur une grande expérience pour déterminer le point particulier de l'écho qui correspond à la position de l'interface. En pratique, l'utilisateur choisit soit l'impulsion de plus grande amplitude, soit l'impulsion centrale du signal d'écho. Rien cependant ne permet de s'assurer que le point choisi correspond effectivement à la position de l'interface. Cette seconde technique permet au plus de déterminer le déplacement d'une paroi relativement rigide, mais ne permet pas de mesurer exactement le diamètre intérieur d'un vaisseau sanguin ou l'épaisseur de la paroi de ce dernier.

L'invention vise à remédier aux inconvénients des techniques connues. Elle consiste essentiellement en une phase d'initialisation dans laquelle on traite un premier signal d'écho pour déterminer la position d'au moins une interface dans ce premier signal d'écho et dans laquelle on suit, en parallèle, la position temporelle d'un ensemble de signaux d'écho acquis postérieurement de manière à déduire la position de l'interface dans le plus récent des signaux d'écho de cet ensemble, lequel survient après la détermination de la position de ladite interface dans ledit premier signal d'écho, et une étape d'acquisition dans laquelle on suit la position de ladite interface.

De manière précise, l'invention a pour objet un procédé de mesure par ultrasons de la position d'au moins une interface mobile consistant à émettre de façon cyclique vers ladite interface une impulsion ultrasonore d'interrogation à une fréquence de répétition Fᵣ, et à recevoir un signal d'écho comportant au moins un écho élémentaire, cet écho élémentaire résultant de la réflexion de ladite impulsion ultrasonore sur ladite interface, ce procédé étant caractérisé en ce qu'il comprend :
- une étape d'initialisation consistant à sélectionner un point de référence dans chaque écho élémentaire d'un ensemble d'échos élémentaires du signal d'écho d'une première impulsion ultrasonore; traiter ledit signal d'écho de ladite première impulsion ultrasonore pour déterminer, dans chacun desdits échos élémentaires, la position temporelle de l'interface ayant produit cet écho élémentaire; calculer, pour chacun desdits échos élémentaires du signal d'écho de ladite première impulsion ultrasonore, l'écart temporel entre la position temporelle du point de référence dudit écho élémentaire et la position temporelle de l'interface obtenue par ledit traitement; et, en parallèle pendant le traitement et le calcul, suivre la position temporelle des points de référence de chacun desdits échos élémentaires d'un premier ensemble de signaux d'écho d'impulsions ultrasonores postérieures à ladite première impulsion ultrasonore;
- une étape d'acquisition consistant à suivre et à mémoriser la position temporelle de l'interface correspondant à chacun desdits échos élémentaires d'un second ensemble de signaux d'écho acquis postérieurement au signal d'écho de ladite première impulsion ultrasonore; et
- une étape d'exploitation des données mémorisées lors de l'étape d'acquisition.

L'invention a également pour objet un appareil pour la mise en oeuvre de ce procédé.

Les caractéristiques et avantages de l'invention ressortiront mieux de la description qui va suivre, faite à titre illustratif mais non limitatif, en référence aux dessins annexés, sur lesquels :
- la figure 1, déjà décrite, illustre le principe de la mesure par ultrasons de la position des interfaces des parois antérieure et postérieure d'un vaisseau sanguin,
- la figure 2, déjà décrite, illustre la forme réelle d'un écho élémentaire produit par l'interface entre un tissu et la paroi d'un vaisseau sanguin,
- la figure 3 représente schématiquement un appareil pour la mise en oeuvre du procédé selon l'invention,
- les figures 4A et 4B sont un organigramme du procédé principal de l'invention et la figure 5 un organigramme du procédé d'interruption, et
- les figures 6 à 9 sont des organigrammes de signaux d'écho illustrant le fonctionnement du procédé de l'invention.

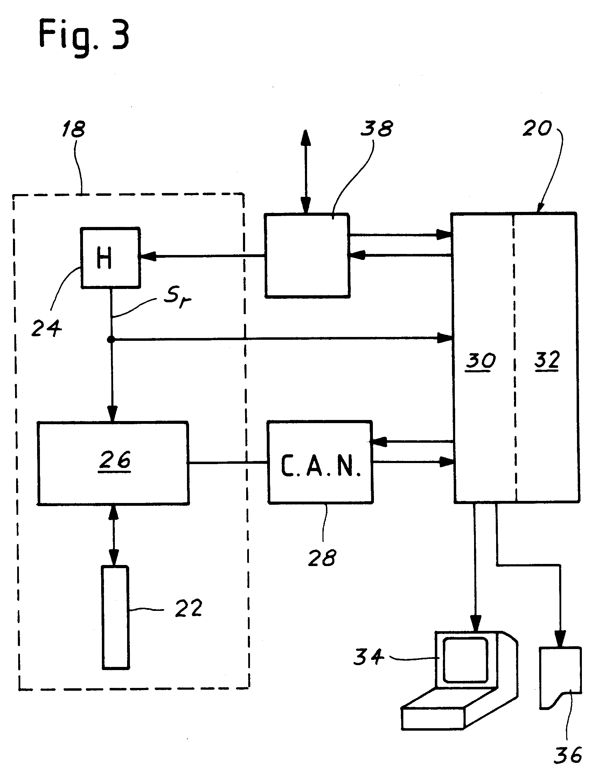

On a représenté schématiquement sur la figure 3 un appareil pour la mise en oeuvre du procédé selon l'invention. Cet appareil comprend principalement un transducteur ultrasonore 18 et un dispositif de calcul 20. Le transducteur ultrasonore 18 comporte une sonde ultrasonore 22 pour émettre un signal ultrasonore et recevoir les échos résultant de la réflexion de cette onde ultrasonore, un circuit de commande 26 pour commander la sonde ultrasonore 22 et une horloge 24. Cette dernière délivre au circuit de commande 26 un signal Sᵣ définissant la fréquence de répétition Fᵣ du signal d'interrogation émis par la sonde ultrasonore.

Le circuit de commande comprend un circuit d'émission délivrant une impulsion électrique qui est transformée par la sonde ultrasonore 22 en un signal ultrasonore correspondant, et un circuit de réception recevant le signal électrique délivré par la sonde ultrasonore correspondant au signal d'écho ultrasonore reçu par la sonde ultrasonore. Le circuit de commande et la sonde ultrasonore sont de type classique. La fréquence centrale de l'impulsion ultrasonore est choisie en fonction de l'application visée. Elle est par exemple de 2 à 20 MHz.

Le signal électrique d'écho délivré par le circuit de commande est reçu par le dispositif de calcul 20 à travers un convertisseur analogique-numérique 28. Pour celui-ci, on peut utiliser un produit du type carte STR 8100 de la société SONIX Inc (Springfield, VA, USA) qui est un convertisseur analogique-numérique 8 bits pouvant traiter jusqu'à 10⁸ échantillons/seconde. Le dispositif de calcul 20 comporte un suiveur d'écho qui est utilisé, de manière classique, pour suivre la position temporelle de chaque écho élémentaire d'un ensemble d'échos élémentaires du signal d'écho par rapport au signal ultrasonore émis. Cette position temporelle, c'est-à-dire finalement le retard de chaque signal d'écho élémentaire sur l'impulsion ultrasonore émise, varie avec la distance entre la sonde ultrasonore et les interfaces mobiles sur lesquelles se réfléchit l'impulsion ultrasonore. Pour réaliser ce suivi, le suiveur d'écho du dispositif de calcul reçoit le signal d'horloge produit par l'horloge 24 et délivre au convertisseur analogique-numérique 28 un signal de retard pour débuter la numérisation du signal d'écho à un instant adéquat. Le suiveur d'écho est de préférence de type détection de l'extremum (positif ou négatif) du signal d'écho numérisé. Cet extremum n'est pas la valeur correcte pour apprécier le mouvement des interfaces mobiles car la distance entre deux points d'échantillonage est égale à c/(2.f), où c ∼ 1500 m/s est la vitesse des ondes ultrasonores dans le milieu et f = 100 MHz est la fréquence d'échantillonnage. Il permet seulement de suivre grossièrement le déplacement de l'écho.

Alternativement, le suiveur d'écho pourrait être de type détection du passage par zéro tel que décrit dans le document EP-A-356629.

Le dispositif de calcul 20 met en oeuvre le procédé de mesure selon l'invention. Il comporte à cet effet principalement un moyen de traitement 30 et un moyen de mémorisation 32. Ce dispositif de calcul est avantageusement un ordinateur individuel à processeur des familles de type 80X86 ou 680X0. Divers équipements périphériques peuvent être ajoutés, tels qu'un moyen d'affichage 34, un moyen d'impression 36 et un circuit d'entrée-sortie 38. Ce dernier peut notamment être relié à l'horloge 24, pour commander depuis le dispositif de calcul la fréquence de répétition Fᵣ du signal d'horloge. Il peut également servir à synchroniser d'autres équipements de mesure tels qu'un sphygmomanomètre, un pléthysmographe ou une sonde Doppler afin de mesurer la pression sanguine et la vitesse du sang.

L'appareil pourrait comprendre une seconde sonde ultrasonore et un second circuit de commande, ce dernier recevant le signal Sᵣ de l'horloge 24. Dans ce cas, le convertisseur analogique-numérique 28 traite alternativement les signaux d'écho reçus par les deux sondes. Ces signaux d'écho étant synchrones, on peut en déduire de manière connue, la vitesse de l'onde de pouls. On pourra se reporter notamment à l'article "Assesment of the true pulse-wave velocity over the physiological pressure range" de Y. Tardy et al. paru Proceeding of the 12th annual international conference of the IEEE engineering in medicine and biology society, Philadelphia, Pennsylvania, Nov. 14, 1990.

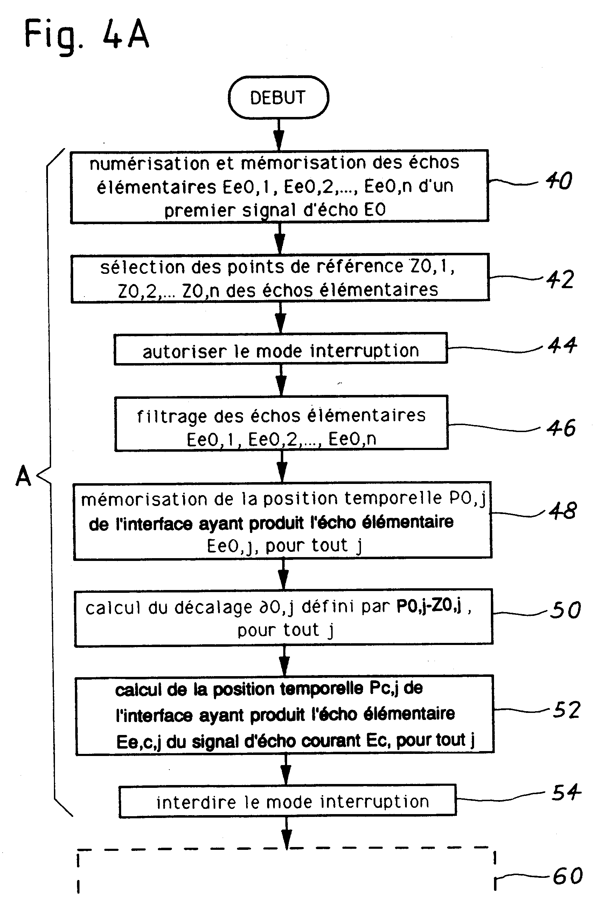

On va maintenant décrire un mode de réalisation du procédé de mesure par ultrasons selon l'invention en référence aux figures 4A à 9.

Avant de débuter la mesure proprement dite, l'utilisateur choisit les paramètres de l'appareil tels que la fréquence de répétition Fᵣ et la sonde, donc la fréquence centrale de l'impulsion ultrasonore. Ces paramètres pourraient également être sélectionnés automatiquement par le dispositif de calcul 20 en fonction de l'application choisie par l'utilisateur. A titre d'exemple, dans le cas de la mesure du diamètre intérieur et de l'épaisseur de la paroi d'un vaisseau sanguin, la fréquence Fᵣ est de l'ordre de 2000 Hz et la fréquence centrale de l'impulsion ultrasonore de l'ordre de 10 MHz pour une mesure de l'artère radiale et de l'ordre de 4 Mhz pour une mesure de la carotide. La durée du retard transmis au convertisseur analogique-numérique 28 de l'appareil est également ajustée, manuellement ou automatiquement, pour que le suiveur d'écho suive correctement chaque écho. Le suiveur d'écho fonctionne ensuite automatiquement. L'utilisateur peut dès lors procéder à la mesure de la position des interfaces du vaisseau sanguin en mettant en oeuvre le procédé de mesure selon l'invention au moyen du dispositif de calcul 20.

Ce procédé de mesure peut être décomposé en trois étapes, à savoir une étape d'initialisation (A), une étape d'acquisition (B) et une étape d'exploitation (C).

L'étape d'initialisation (A) consiste à déterminer la position temporelle des interfaces mobiles ayant provoqué chaque écho élémentaire d'un premier signal d'écho. A cette fin, le signal d'écho E₀ produit par une impulsion ultrasonore est numérisé par le convertisseur analogique-numérique 28 et les échos élémentaires Ee_{0,1}, Ee_{0,2},...,Ee_{0,n} correspondant chacun à la réflexion de l'impulsion ultrasonore sur une interface mobile sont mémorisés dans le moyen de mémorisation 32 (opération 40). En pratique, on mémorise les parties du signal d'écho qui correspondent aux échos des parois antérieure E_{ant,0} et postérieur E_{post,0} du vaisseau sanguin. Chacun de ces échos peut comporter un ou plusieurs échos élémentaires, qui seront révélés après traitement du signal. Dans le cas d'un vaisseau sanguin, chaque impulsion ultrasonore produit normalement quatre échos élémentaires, deux de ces échos élémentaires étant produits par la paroi antérieure, alors que les deux autres échos élémentaires sont produits par la paroi postérieure.

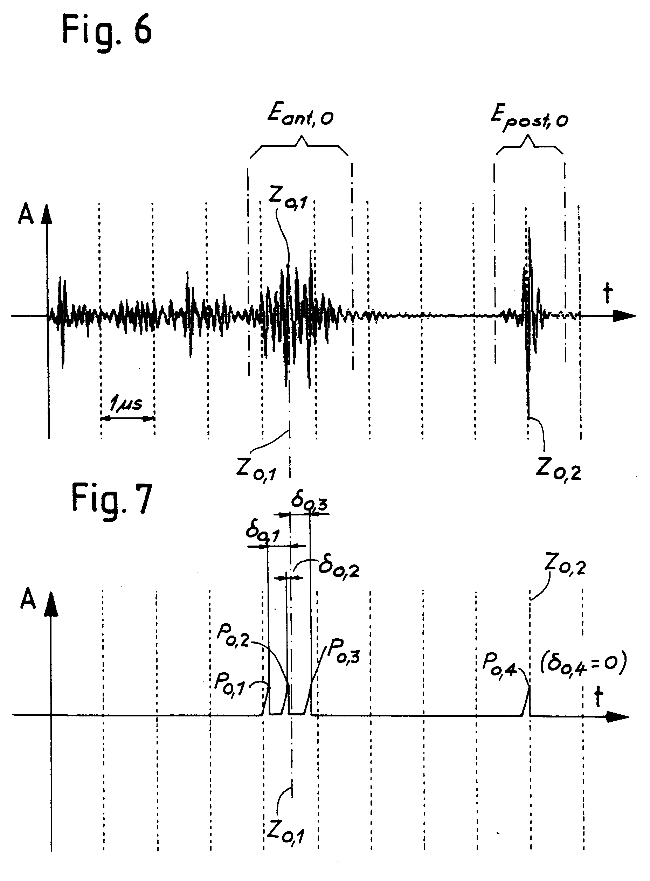

Le dispositif de calcul 20 sélectionne ensuite dans chaque écho élémentaire un point de référence destiné à identifier la position temporelle de chaque écho élémentaire (opération 42 et figure 6). Ces points de référence Z_{0,i}, où 0≦i≦m, sont de préférence un point remarquable de chaque écho élémentaire, tel que l'impulsion de plus grande amplitude ou l'impulsion centrale de l'écho élémentaire. Lorsque deux échos élémentaires se déplacent de la même manière en fonction du temps, il est possible de marquer la position temporelle de ces deux échos élémentaires à l'aide d'un unique point de référence. C'est le cas, en particulier, des échos élémentaires produits respectivement par la paroi antérieure et la paroi postérieure d'un vaisseau sanguin. On peut donc dans ce cas choisir un seul point de référence Z_{0,1}, resp. Z_{0,2} pour l'ensemble des échos élémentaires de l'écho E_{ant,0}, resp. E_{post,0} comme cela est représenté sur les figures 6 et 7.

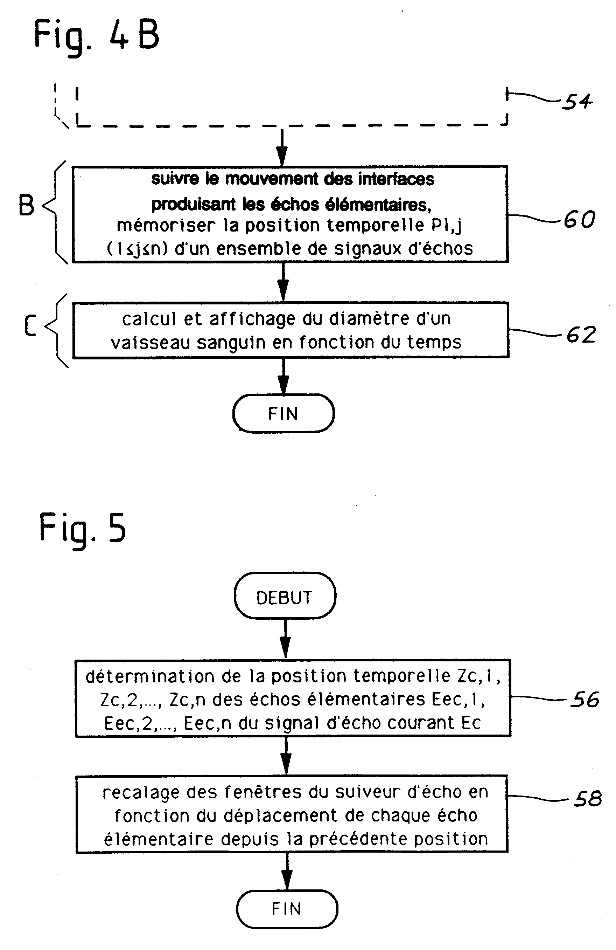

La position temporelle de l'interface mobile correspondant à chaque écho élémentaire d'un ensemble d'échos élémentaires sélectionnés dans ledit premier signal d'écho est ensuite déterminée à partir du signal d'écho élémentaire numérisé et mémorisé. Cette détermination consiste en un traitement du signal numérisé pour en éliminer le bruit et extraire ainsi l'impulsion résultant de la réflexion de l'impulsion ultrasonore sur l'interface mobile. Pendant ce traitement, dont la durée est de l'ordre de quelques secondes avec les moyens de traitement actuels, il faut continuer à suivre la position des échos élémentaires. Ceci nécessite de recourir à un dispositif de calcul pouvant effectuer ces deux tâches en parallèle ou de procéder par interruption périodique du traitement pour suivre le déplacement des échos élémentaires. C'est cette seconde technique qui est représentée sur les figures 4 et 5.

Différentes techniques de traitement sont connues pour déterminer la position d'une interface mobile à partir de l'écho produit par la réflexion d'une impulsion ultrasonore sur cette interface mobile. A titre d'exemple, on peut utiliser la technique de traitement décrite dans le document EP-A-409054.

L'opération de traitement 46 permet d'obtenir à partir de chaque écho élémentaire une impulsion qui marque la position temporelle P_{0,j} de l'interface mobile correspondante (cf. figure 7). Dans l'exemple représenté, trois interfaces ont été détectées dans l'écho E_{ant,0} de la paroi antérieure du vaisseau sanguin (ce sont respectivement l'interface tissu-vaisseau, une interface interne du vaisseau et l'interface vaisseau-sang), alors qu'une seule interface est visible dans l'écho E_{post,0} de la paroi postérieure du vaisseau sanguin. Le dispositif de calcul procède ensuite à la mémorisation de ces positions temporelles P_{0,j} pour chaque écho élémentaire Ee_{0,j} de l'écho E₀ (opération 48) et calcule l'écart temporel δ_{0,j} entre la position temporelle du point de référence Z_{0,i} et la position temporelle P_{0,j} de l'interface (opération 50). Cet écart temporel reste le même pour les échos élémentaires successifs produits par une même interface mobile en réponse à des impulsions ultrasonores successives. On note que le point de référence Z_{0,1} choisi pour suivre la position temporelle de l'écho E_{ant,0} ne correspond à aucune interface mobile, alors que le point de référence Z_{0,2} choisi pour suivre la position temporelle de l'écho E_{post,0} se trouve correspondre, par hasard, à la position de l'interface mobile détectée.

La connaissance de l'écart temporel pour chaque écho élémentaire permet de déterminer, dans le signal d'écho élémentaire non filtré, l'impulsion marquant la position de l'interface mobile, à partir de la position du point de référence. Ceci permet de recaler le suiveur d'écho sur l'impulsion correspondant à la position effective de chaque interface mobile (opération 52). Il est également possible de continuer à caler le suiveur d'écho sur le point de référence de chaque écho élémentaire, étant entendu que la position de chaque interface mobile s'en déduit immédiatement avec l'écart temporel.

Pendant les opérations 46 à 52, le traitement effectué par le dispositif de calcul doit être interrompu périodiquement pour assurer le suivi du déplacement des échos élémentaires. Les opérations 44 et 54 placent respectivement le dispositif de calcul en mode INTERRUPTION AUTORISEE et en mode INTERRUPTION INTERDITE.

Le traitement est interrompu à une fréquence suffisante pour que le suivi d'écho soit assuré. Cette fréquence d'interruption Fᵢ dépend de la vitesse maximale de déplacement des parois et de la fréquence Fᵣ de l'impulsion ultrasonore d'interrogation. Il faut également que cette fréquence d'interruption Fᵢ ne soit pas trop élevée pour que le traitement ne soit pas interrompu trop souvent. Dans le cas de la mesure sur un vaisseau sanguin, une fréquence d'interruption Fᵢ de quelques centaines de hertz peut être utilisée. De manière avantageuse, le signal d'interruption est obtenu par division du signal de répétition délivré par l'horloge 24. Le suiveur d'écho reçoit alors le signal d'écho d'une impulsion ultrasonore sur k, où k est le rapport entre la fréquence de répétition Fᵣ et la fréquence d'interruption Fᵢ.

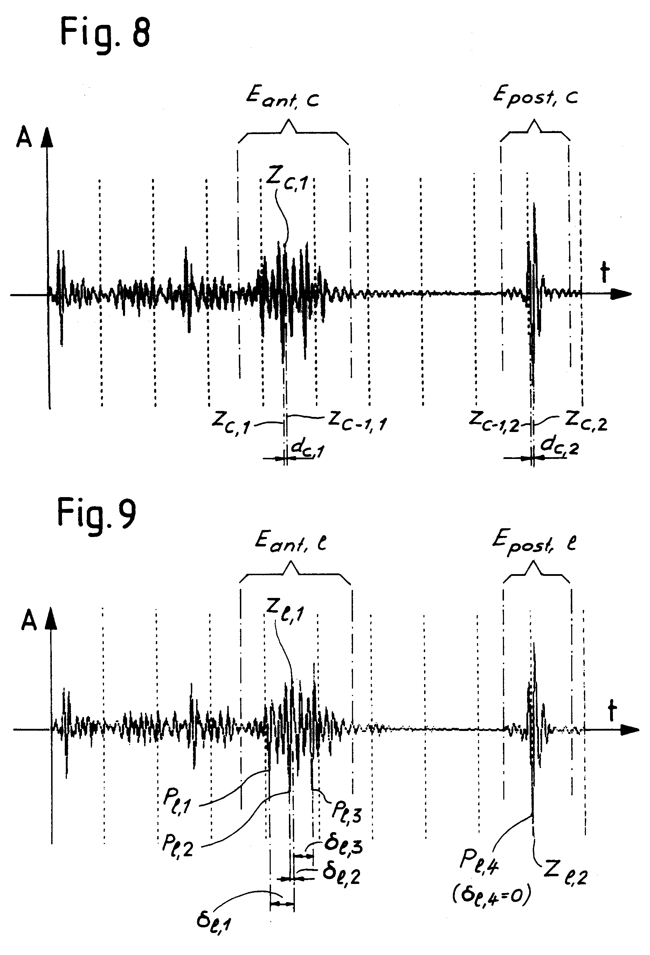

Le traitement d'interruption comprend une opération 56 de détermination de la position temporelle des points de référence Z_{c,i} des échos élémentaires Ee_{c,j} du premier signal d'écho E_{c} reçu après le signal d'interruption, ou, ce qui revient au même, de mesure du déplacement de chaque point de référence entre le signal d'écho E_{c} reçu après le signal d'interruption et le précédent signal d'écho E_{c-1}, et une opération 58 de recalage des fenêtres du suiveur d'écho en fonction du déplacement d_{c,1}, d_{c,2} , de chaque écho élémentaire depuis le dernier signal d'interruption (cf. figure 8). Ces opérations étant classiques dans le domaine de la mesure par ultrasons de la position d'une paroi mobile, il n'y a pas lieu de les décrire plus en détail ici.

L'étape d'initialisation qui vient d'être décrite est suivie de l'étape d'acquisition (B). Cette étape comporte une opération 60 consistant à suivre la position temporelle de chaque interface mobile dudit ensemble d'échos élémentaires sélectionnés dans ledit premier signal d'écho. Pour ce faire, on note les positions Pᵢ indiquées par le suiveur d'écho et on procède à une interpolation quadratique avec les points d'échantillonnage voisins. On obtient alors une précision de l'ordre de 0,5 µm (avec une fréquence d'échantillonnage de 100 MHz). Ces positions sont mémorisées pour un ensemble déterminé d'impulsions ultrasonores. De préférence, l'acquisition se fait sur un ensemble d'impulsions ultrasonores reçues après la fin de l'étape d'initialisation. Cependant, il est possible de choisir un autre ensemble d'impulsions ultrasonores, et par exemple de retenir, pour l'étape d'acquisition, certaines ou toutes les impulsions ultrasonores mémorisées en réponse au signal d'interruption pendant l'étape d'initialisation.

De manière classique, pour augmenter la résolution, il est avantageux d'acquérir les ondes ultrasonores à une première fréquence (en pratique, cette première fréquence est simplement la fréquence de répétition Fᵣ ), et de ne mémoriser les ondes ultrasonores, ou au moins la position de chaque interface mobile, qu'à une seconde fréquence, sous-multiple de la première fréquence, la position mémorisée de chaque interface mobile étant égale à la moyenne des positions relevées au cours des p dernières ondes ultrasonores, où p est le rapport entre la première fréquence et le seconde fréquence. La précision sur la position des interfaces est ainsi accrue d'un facteur √p.

En ce qui concerne la mémorisation des données relatives à chaque écho élémentaire Ee_{l,j}, on peut choisir la position temporelle de l'interface mobile P_{l,j} ou la position temporelle du point de référence Z_{l,i}, la différence entre ces deux positions étant, pour chaque écho élémentaire, constante et égale à l'écart temporel δ_{0,j} déterminé lors de l'opération 50 (cf. figure 9). De même, comme indiqué plus haut en référence à l'opération 52, le suiveur d'écho peut être calé soit sur la position des points de référence, soit sur la position des interfaces mobiles.

Le volume de données mémorisées dépend de la mémoire disponible dans le dispositif de calcul 20 et du temps alloué à l'étape d'acquisition.

Il ne reste plus ensuite qu'à exploiter les données mémorisées en fonction des besoins de l'utilisateur (opération 62). Cette exploitation (C) peut consister à calculer et à visualiser le diamètre intérieur et l'épaisseur de la paroi du vaisseau sanguin en fonction du temps, et à déduire d'autres paramètres physiologiques tels que la compliance du vaisseau sanguin en combinant les données relatives au diamètre avec des données relatives au flux sanguin obtenues à l'aide d'autres appareils de mesure, par exemple des données de pression sanguine à l'endroit de la mesure effectuée dans le cadre de la présente invention.

## Revendications

1. Procédé de mesure par ultrasons de la position d'au moins une interface mobile consistant à émettre de façon cyclique vers ladite interface une impulsion ultrasonore d'interrogation à une fréquence de répétition Fᵣ, et à recevoir un signal d'écho (E_{c}) comportant au moins un écho élémentaire (Ee_{c,j}), cet écho élémentaire résultant de la réflexion de ladite impulsion ultrasonore sur ladite interface, ledit procédé étant caractérisé en ce qu'il comprend :
- une étape d'initialisation consistant à sélectionner un point de référence (Z_{0,i}) dans chaque écho élémentaire d'un ensemble d'échos élémentaires (Ee_{0,i}) du signal d'écho (E₀) d'une première impulsion ultrasonore; traiter ledit signal d'écho de ladite première impulsion ultrasonore pour déterminer, dans chacun desdits échos élémentaires, la position temporelle (P_{0,j}) de l'interface ayant produit cet écho élémentaire; calculer, pour chacun desdits échos élémentaires du signal d'écho de ladite première impulsion ultrasonore, l'écart temporel (δ_{0,j}) entre la position temporelle du point de référence dudit écho élémentaire et la position temporelle de l'interface obtenue par ledit traitement; et, en parallèle pendant le traitement et le calcul, suivre la position temporelle (Z_{c,i}) des points de référence de chacun desdits échos élémentaires (Ee_{c,j}) d'un premier ensemble de signaux d'écho d'impulsions ultrasonores postérieures à ladite première impulsion ultrasonore;
- une étape d'acquisition consistant à suivre et à mémoriser la position temporelle (P_{c,j}) de l'interface correspondant à chacun desdits échos élémentaires d'un second ensemble de signaux d'écho acquis postérieurement au signal d'écho de ladite première impulsion ultrasonore; et
- une étape d'exploitation des données mémorisées lors de l'étape d'acquisition.

2. Procédé selon la revendication 1, caractérisé en ce que l'impulsion de plus grande amplitude d'un écho élémentaire est sélectionnée comme point de référence de cet écho élémentaire.

3. Procédé selon la revendication 1, caractérisé en ce que l'impulsion centrale d'un écho élémentaire est sélectionnée comme point de référence de cet écho élémentaire.

4. Procédé selon l'une quelconque des revendications 1 à 3, caractérisé en ce que l'ensemble de signaux d'écho suivi pendant l'étape d'initialisation est un sous-ensemble strict de l'ensemble des signaux d'écho reçus à la fréquence de répétition Fᵣ.

5. Procédé selon la revendication 4, pour la mesure de la position des interfaces externes et internes des parois antérieure et postérieure d'un vaisseau sanguin par émission d'une onde ultrasonore suivant un diamètre dudit vaisseau sanguin, caractérisé en ce que, pendant l'étape d'initialisation, un seul et même point de référence est choisi pour suivre les positions des interfaces externe et interne d'une même paroi.

6. Procédé selon l'une quelconque des revendications 1 à 5, caractérisé en ce que lorsque le traitement de l'étape d'initialisation est terminé, on déduit des écarts temporels calculés la position temporelle de l'interface correspondant à chacun desdits échos élémentaires du plus récent signal d'écho suivi, à partir de la position temporelle du point de référence de chacun desdits échos élémentaires, et en ce que, lors de l'étape d'acquisition, le second ensemble de signaux d'écho acquis est postérieur audit plus récent signal d'écho.

7. Procédé selon l'une quelconque des revendications 1 à 5, caractérisé en ce qu'au moins une partie des signaux d'écho du second ensemble est formée de signaux d'écho du premier ensemble.

8. Appareil de mesure par ultrasons de la position d'au moins une interface mobile pour la mise en oeuvre du procédé selon la revendication 1, comprenant un transducteur ultrasonore (18) pour émettre, de façon cyclique, vers ladite interface une impulsion ultrasonore d'interrogation à une fréquence de répétition Fᵣ, et pour recevoir un signal d'écho comportant au moins un écho élémentaire, résultant de la réflexion de ladite impulsion ultrasonore sur ladite interface, ledit appareil étant caractérisé en ce qu'il comprend des moyens de traitement (30) et des moyens de mémorisation (32) conçus pour exécuter ladite étape d'initialisation dudit procédé.

9. Appareil selon la revendication 8, caractérisé en ce que le transducteur ultrasonore (18) comprend une sonde ultrasonore (22) de type émetteur-récepteur, un circuit de commande (24) de ladite sonde ultrasonore, et une horloge (26) pour cadencer l'émission des impulsions ultrasonores, ledit circuit de commande délivrant audit moyen de traitement un signal électrique représentatif du signal d'écho ultrasonore reçu par la sonde ultrasonore.

10. Appareil selon la revendication 8 ou 9, caractérisé en ce qu'il comprend un convertisseur analogique-numérique (28) entre le circuit de commande (24) et le moyen de traitement (30).

11. Appareil selon l'une des revendications 8 à 10, caractérisé en ce que le moyen de traitment (30) comporte une entrée d'interruption recevant un signal d'interruption d'un circuit d'interruption, le moyen de traitement interrompant alors l'opération en cours pour mémoriser la position temporelle des points de référence de chacun desdits échos élémentaires du signal d'écho reçu immédiatement après ladite interruption.

12. Appareil selon la revendication 11, caractérisé en ce que ledit circuit d'interruption est une horloge, la fréquence du signal délivré par ladite horloge étant un sous-multiple de la fréquence de répétition Fᵣ.

13. Appareil selon l'une quelconque des revendications 8 à 12, caractérisé en ce qu'il comprend des moyens de présentation (34, 36) des données mémorisées et de résultats de ladite étape d'exploitation.

## Claims

1. Process for ultrasonically measuring the position of at least one mobile interface consisting of transmitting an ultrasonic interrogation impulse at a repetition frequency Fᵣ, and in receiving an echo signal (E_{c}) having at least one elemental echo (Ee_{c,j}), this elemental echo resulting from the reflection of said ultrasonic impulse onto said interface, said process being characterized in that it comprises :
- an initialization stage consisting in selecting a reference point (Z_{0,i}) in each elemental echo of a group of elemental echoes (Ee_{0,i}) of the echo signal (E₀) of a first ultrasonic impulse; processing said echo signal of said first ultrasonic impulse to determine, for each of said elemental echoes, the temporal position (P_{0,j}) of the interface which produced this elemental echo; calculating, for each of said elemental echoes of the echo signal of said first ultrasonic impulse, the temporal interval (δ_{0,j}) between the temporal position of the reference point of said elemental echo and the temporal position of the interface obtained by said processing; and, simultaneously during the processing and calculation, tracking the temporal position (Z_{c,i}) of the reference points of each of said elemental echoes (Ee_{c,j}) of a first group of echo signals of ultrasonic impulses after said first ultrasonic impulse;
- an assimilation phase consisting in tracking and memorizing the temporal position (P_{c,j}) of the interface corresponding to each of said elemental echoes of a second group of echo signals acquired subsequent to the echo signal of said first ultrasonic impulse; and
- a processing stage of the data memorized during the assimilation phase.

2. Process according to claim 1, characterized in that the impulse of the largest amplitude of an elemental echo is selected as the reference point of this elemental echo.

3. Process according to claim 1, characterized in that the central impulse of an elemental echo is selected as the reference point of this elemental echo.

4. Process according to any one of claims 1 to 3, characterized in that the group of echo signals tracked during the initialization phase is a precise sub-group of the group of echo signals received at the repetition frequency Fᵣ.

5. Process according to claim 4 for measuring the position of the external and internal interfaces of the anterior and posterior walls of a blood vessel by emission of an ultrasonic wave tracking one diameter of said blood vessel, characterized in that, during the initialization phase, one and the same reference point is chosen to follow the positions of the external and internal interfaces of the same wall.

6. Process according to any one of claims 1 to 5, characterized in that, when the initialization phase is completed, the temporal position of the interface corresponding to each of said elemental echoes of the most recent echo signal tracked is subtracted from the temporal calculated intervals from the temporal position of the reference point of each of said elemental echoes, and in that, during the assimilation stage, the second group of echo signals assimilated is subsequent to said most recent echo signal.

7. Process according to any one of claims 1 to 5, characterized in that at least one part of the echo signals of the second group is composed of echo signals of the first group.

8. Apparatus for measuring using ultrasound the position of at least one mobile interface for carrying out the process according to claim 1, comprising an ultrasonic transducer (18) to emit towards said interface, in cyclical manner, an ultrasonic interrogation impulse at a repetition frequency Fᵣ, and to receive an echo signal having at least one elemental echo, resulting from the reflection of said ultrasonic impulse on said interface, said apparatus being characterized in that it comprises processing means (30) and memorizing means (32) designed to carry out said initialization stage of said process.

9. Apparatus according to claim 8, characterized in that the ultrasonic transducer (18) comprises an ultrasonic probe (22) of the emitter-receiver type, a control circuit (24) for said ultrasonic probe and a clock (26) to control the emission of the ultrasonic impulses, said control circuit delivering to said processing means an electrical signal representative of the ultrasonic echo signal received by the ultrasonic probe.

10. Apparatus according to claim 8 or 9, characterized in that it comprises an analog-digital converter (28) between the control circuit (24) and the processing means (30).

11. Apparatus according to one of claims 8 to 10, characterized in that the processing means (30) has an interrupt input receiving an interrupt signal from an interrupt circuit, the processing means then interrupting the operation in process to memorize the temporal position of the reference points of each of said elemental echoes of the echo signal received immediately after said interruption.

12. Apparatus according to claim 11, characterized in that said interrupt circuit is a clock, the frequency of the signal delivered by said clock being a sub-multiple of the repetition frequency Fᵣ.

13. Apparatus according to any one of claims 8 to 12, characterized in that it comprises means (34, 36) for presenting the data memorized and the results of said operational stage.

## Patentansprüche

1. Verfahren zur Ultraschallmessung der Position mindestens einer beweglichen Grenzfläche, bestehend aus der zyklischen Aussendung in Richtung der Grenzfläche eines Ultraschallabfrageimpulses mit einer Wiederholungsfrequenz Fᵣ und dem Auffangen eines Echosignals (E_{c}), das mindestens ein Elementarecho (Ee_{c,j}) umfaßt, welches Elementarecho herrührt von der Reflektion des Ultraschallimpulses an der Grenzfläche, welches Verfahren dadurch gekennzeichnet ist, daß es umfaßt:
- eine Initialisierungsetappe, bestehend aus der Auswahl eines Referenzpunktes (Z_{0,i}) in jedem Elementarecho einer Gruppe von Elementarechos (Ee_{0,i}) des Echosignals (E₀) eines ersten Ultraschallimpulses; der Verarbeitung des genannten Echosignals des genannten ersten Ultraschallimpulses zum Bestimmen, in jedem der Elementarechos, der zeitlichen Position (P_{0,j}) der Grenzfläche, welche dieses Elementarecho hervorgerufen hatte; dem Berechnen für jedes der Elementarechos des Echosignals des ersten Ultraschallimpulses des zeitlichen Versatzes (δ_{0,j}) zwischen der zeitlichen Position des Referenzpunktes dieses Elementarechos und der zeitlichen Position der Grenzfläche, erhalten durch die genannte Verarbeitung; und, parallel während der Verarbeitung und der Berechnung der Verfolgung der zeitlichen Position (Z_{c,i}) von Referenzpunkten jedes Elementarechos (Ee_{c,j}) einer ersten Gruppe von Echosignalen von Ultraschallimpulsen nach dem ersten Ultraschallimpuls;
- eine Akquisitionsetappe, bestehend aus der Verfolgung und Abspeicherung der zeitlichen Position (P_{c,j}) der Grenzfläche entsprechend jedem der Elementarechos an einer zweiten Gruppe von Echosignalen, gewonnen nach dem Echosignal des ersten Ultraschallimpulses; und
- eine Auswertungsetappe der während der Akquisitionsetappe abgespeicherten Daten.

2. Verfahren nach Anspruch 1, dadurch gekennzeichnet, daß der Impuls größter Amplitude eines Elementarechos als Referenzpunkt dieses Elementarechos gewählt wird.

3. Verfahren nach Anspruch 1, dadurch gekennzeichnet, daß der zentrale Impuls eines Elementarechos als Referenzpunkt dieses Elementarechos ausgewählt wird.

4. Verfahren nach einem der Ansprüche 1 bis 3, dadurch gekennzeichnet, daß die Gruppe von Echosignalen, die während der Initialisierungsetappe verfolgt wird, eine strikte Untergruppe der Gruppe von Echosignalen ist, empfangen mit der Wiederholungsfrequenz Fᵣ.

5. Verfahren nach Anspruch 4 für die Messung der Position von äußeren und inneren Grenzflächen von vorderen bzw. hinteren Wandungen eines Blutgefäßes durch Aussendung einer Ultraschallwelle in Richtung auf einen Durchmesser des Blutgefäßes, dadurch gekennzeichnet, daß während der Initialisierungsetappe ein einziger und gleicher Referenzpunkt für die Verfolgung der Positionen dieser äußeren und inneren Grenzflächen ein- und derselben Wandung gewählt wird.

6. Verfahren nach einem der Ansprüche 1 bis 5, dadurch gekennzeichnet, daß nach Abschluß der Verarbeitung in der Initialisierungsetappe man aus den berechneten zeitlichen Versätzen die zeitliche Position der Grenzfläche ableitet, entsprechend jedem der Elementarechos des jüngsten verfolgten Echosignals, ausgehend von der zeitlichen Position des Referenzpunktes jedes der Elementarechos und daß während der Akquisitionsetappe die zweite Gruppe von gewonnenen Echosignalen später ist als das genannte jüngste Echosignal.

7. Verfahren nach einem der Ansprüche 1 bis 5, dadurch gekennzeichnet, daß mindestens eine Partie der Echosignale der zweiten Gruppe aus Echossignalen der ersten Gruppe gebildet wird.

8. Ultraschallmeßvorrichtung für die Position mindestens einer beweglichen Grenzfläche für die Ausführung des Verfahrens nach Anspruch 1, umfassend einen Ultraschallwandler (18) für die zyklische Aussendung in Richtung der Grenzfläche eines Abfrageultraschallimpulses mit einer Wiederholungsfrequenz Fᵣ und für den Empfang eines Echosignals, umfassend mindestens ein Elementarecho, herrührend von der Reflektion des Ultraschallimpulses an der Grenzfläche, welche Vorrichtung dadurch gekennzeichnet ist, daß sie Verarbeitungsmittel (30) und Speichermittel (32) umfaßt, konzipiert für die Ausführung der Initialisierungsetappe des Verfahrens.

9. Vorrichtung nach Anspruch 8, dadurch gekennzeichnet, daß der Ultraschallwandler (18) eine Ultraschallsonde (22) vom Typ Sender-Empfänger, einen Steuerschaltkreis (24) der Ultraschallsonde und einen Taktgeber (26) für die Kadenzbildung der Aussendung von Ultraschallimpulsen umfaßt, wobei der Steuerschaltkreis dem Verarbeitungsmittel ein elektrisches Signal zuführt, das repräsentativ ist für das Ultraschallechosignal, empfangen von der Ultraschallsonde.

10. Vorrichtung nach Anspruch 8 oder 9, dadurch gekennzeichnet, daß sie einen Analog-Digital-Umsetzer (28) zwischen dem Steuerschaltkreis (24) und den Verarbeitungsmitteln (30) umfaßt.

11. Vorrichtung nach einem der Ansprüche 8 bis 10, dadurch gekennzeichnet, daß die Verarbeitungsmittel (30) einen unterbrechereingang umfassen, der ein Unterbrechersignal einer Unterbrecherschaltung empfängt, wobei die Verarbeitungsmittel so den laufenden Betrieb unterbrechen für die Abspeicherung der zeitlichen Position von Referenzpunkten jedes der Elementarechos des unmittelbar nach der Unterbrechung empfangenen Echosignals.

12. Vorrichtung nach Anspruch 11, dadurch gekennzeichnet, daß die Unterbrecherschaltung ein Taktgeber ist, wobei die Wiederholungsfrequenz Fᵣ ein Vielfaches der Signalfrequenz, abgegeben von dem Taktgeber, ist.

13. Vorrichtung nach einem der Ansprüche 8 bis 12, dadurch gekennzeichnet, daß sie Präsentationsmittel (34, 36) der abgespeicherten Daten und der Resultate der Auswertungsetappe umfaßt.
